# EUROPEAN PATENT APPLICATION

(11) **EP 4 834 355 A1**
(43) Date of publication of application: **04.11.2026**
(21) Application number: 24912811.7
(22) Date of filing: 24.12.2024
(51) Int. Cl.: A61K 36/02, A23L 33/135, A61K 8/9706, A61K 8/9722, A61K 36/05, A61K 47/10, A61Q 19/00

(54) **COMPOSITION CONTAINING EXTRACELLULAR VESICLES DERIVED FROM MICROALGAE**

(30) Priority: 27.12.2023 JP 2023221201
(71) Applicant: Rohto Pharmaceutical Co., Ltd., Osaka-shi, Osaka 544-8666 (JP)
(72) Inventor: OKADA, Keita, Osaka-shi, Osaka 544-8666 (JP); MASUDA, Keiko, Osaka-shi, Osaka 544-8666 (JP); MATSUMOTO, Nozomi, Osaka-shi, Osaka 544-8666 (JP)
(74) Representative: Cabinet Beau de Loménie
(86) International application number: PCT/JP2024/045689
(87) International publication number: WO 2025/142924

(57) **Abstract**

An object of the present invention is to provide a composition containing extracellular vesicles derived from microalgae. In one aspect of the present invention, a composition containing: microalgae-derived extracellular vesicles (A); and optionally one type or two or more types selected from a group consisting of (B-1) to (B-3) is prepared: (B-1) one type or two or more types selected from a group consisting of a primary alcohol and a polyhydric alcohol; (B-2) one type or two or more types selected from a group consisting of an organic acid and a salt thereof; and (B-3) a chelating agent.

## Description

### TECHNICAL FIELD

The present invention relates to a composition containing extracellular vesicles derived from microalgae.

### BACKGROUND ART

Many cell types extracellularly secrete vesicles having a lipid bilayer membrane. Such vesicles are referred to as extracellular vesicles, and typically contain functional molecules such as proteins and nucleic acids within the lipid bilayer membrane. Extracellular vesicles are involved in the transfer of functional molecules in living organisms, and have recently been found to be involved in various biological phenomena and diseases.

For example, it is known that extracellular vesicles produced by lactic acid bacteria of a specific bacterial species are used for preventing hair loss and promoting hair growth (Patent Document 1).

### PRIOR ART DOCUMENT

### PATENT DOCUMENT

Patent Document 1: JP-A-2018-529720

### SUMMARY OF THE INVENTION

### PROBLEMS TO BE SOLVED BY THE INVENTION

However, there are many unknown aspects of extracellular vesicles, and in particular, there are no sufficient reports on microalgae-derived extracellular vesicles themselves and applications thereof.

Therefore, an object of the present invention is to provide a composition containing extracellular vesicles derived from microalgae and having excellent stability of the extracellular vesicles.

### MEANS FOR SOLVING THE PROBLEMS

In order to solve the above problems, the present inventors have extensively conducted studies, and resultantly found that a composition containing extracellular vesicles derived from microalgae has excellent stability compared to extracellular vesicles derived from other sources, and also has good properties in combination with a base, thereby completing the present invention.

That is, the present invention provides a composition containing microalgae-derived extracellular vesicles (A).

One aspect of the present invention is as follows.
[1] A composition containing microalgae-derived extracellular vesicles (A).

### EFFECT OF THE INVENTION

According to the present invention, a composition containing extracellular vesicles derived from microalgae and having excellent stability can be obtained.

### MODE FOR CARRYING OUT THE INVENTION

### [Composition containing extracellular vesicles]

The composition of the present invention contains microalgae-derived extracellular vesicles (A). The composition may further contain one type or two or more types selected from a group consisting of (B-1) to (B-3):
(B-1) one type or two or more types selected from a group consisting of a primary alcohol and a polyhydric alcohol;
(B-2) one type or two or more types selected from a group consisting of an organic acid and a salt thereof; and
(B-3) a chelating agent.

### (Extracellular vesicles)

The extracellular vesicles derived from microalgae in the present description are typically those contained in a culture supernatant of microalgae, and in the preparation of a composition containing extracellular vesicles, the culture supernatant can be used as it is, or can be used after being roughly purified or purified from the culture supernatant. The composition is a preparation obtained from a culture supernatant and may contain a secretion obtained by culture in addition to the extracellular vesicles.

### (Culture supernatant of microalgae)

In the present description, "microalgae" refer to organisms having a cell size of 0.1 µm to 1000 µm in diameter among the rest of organisms performing photosynthesis other than mosses, ferns, and seed plants that generate oxygen. Also included are labyrinthulas, which are protists closely related to microalgae.

Examples of the microalgae include organisms of prokaryotes such as Cyanobacteria, and eukaryotes such as Glaucophyta, Rhodophyta (red algae), Chlorophyta, Cryptophyta, Haptophyta, Heterokontophyta, Dinophyta, Euglenida, and Chlorarachniophyta.

Examples of the microalgae include microalgae belonging to the genus Pavlova, Euglena (Protozoa/green algae), Spirulina (blue-green algae), Chlorella (green algae), Dunaliella (green algae), Nannochloropsis (eustigmatophyceans), Scenedesmus (green algae), Botryococcus (green algae), and the Thraustochytrid of Labyrinthula. The microalgae exemplified above may be used alone or in combination of two or more thereof.

Among the microalgae, from the viewpoint of remarkably exhibiting the effect of the present invention, microalgae belonging to at least one species selected from the group consisting of the genus Pavlova, Euglena, Spirulina, and Chlorella are preferable, microalgae belonging to at least one species selected from the group consisting of the genus Pavlova, Euglena, and Spirulina are more preferable, microalgae belonging to at least one species selected from the group consisting of the genus Pavlova and Euglena are still more preferable, and microalgae belonging to the genus Pavlova are particularly preferable.

Pavlova is included in the phylum of the Haptophyta, and examples of the microalgae of the genus Pavlova include P. calceolate, P. granifera, P. gyrans, P. lutheri, P. pinguis, and P. salina. Among them, P. granifera and/or P. gyrans are preferable.

Examples of the microalgae of the genus Euglena include E. gracilis, E. longa, E. caudata, E. oxyuris, E. tripteris, E. proxima, E. viridis, E. sociabilis, E. ehrenbergii, E. deses, E. pisciformis, E. spirogyra, E.acus, E. geniculata, E. intermedia, E. mutabilis, E. sanguinea, E. stellata, E. terricola, E. klebsi, E. rubra, and E. cyclopicola. Among them, E. gracilis and/or E. longa are preferable.

The microalgae of the genus Spirulina may be, for example, the genus Arthrospira, a scientific name changed from the genus Spirulina, and examples thereof include S. platensis (A. platensis) and S. maxima (A. maxima). Among them, S. platensis is preferable.

Examples of the microalgae of the genus Chlorella include C. vulgaris, C. saccharophila, C. ellipsoidea, C. pyrenoidosa, C. sorokiniana, and C. lobophora. Among them, C. vulgaris is preferable.

The microalgae exemplified in the present invention are classified as lower organisms and share common characteristics with respect to the lipids contained in their cells. Examples of the lipids commonly contained in these microalgae include so-called betaine lipids. Betaine lipids are lipids having a unique structure different from phospholipids and the like generally found in higher organisms. Furthermore, it is considered that betaine lipids commonly contained in microalgae also contribute to lipids constituting the membrane structure of extracellular vesicles secreted by these microalgae. For this reason, it is presumed that the membrane of the extracellular vesicles has common membrane characteristics reflecting the properties unique to microalgae. Therefore, the effects of the invention of the present application are expected regardless of which extracellular vesicles derived from microalgae are used.

The microalgae exemplified above are widely distributed in sea water, fresh water such as in ponds and marshes, and brackish water. The microalgae may be separated from the water and then used. Alternatively, any microalgae already isolated may be used.

The microalgae exemplified above include related species and mutant strains thereof as long as the effect of the present invention is exhibited. Examples of the mutant strain include those obtained by a genetic method such as genetic recombination, transduction, or transformation.

In the present description, the culture supernatant of microalgae refers to a supernatant of a culture solution after microalgae are cultured. Examples of the supernatant include a supernatant of a culture solution obtained by culturing microalgae using a known culture method, and removing the microalgae using a known separation means such as centrifugation.

Culture conditions for preparing the culture supernatant of microalgae are not particularly limited as long as the effect of the present invention is exhibited, and a known method can be used. The following examples are given.

### (Culture conditions)

The culture of microalgae can be performed using a culture solution. A carbon source is added to the culture solution as a nutrient source. As the carbon source, an inorganic carbon source (CO₂, NaHCO₃, Na₂CO₃, or the like), an organic carbon source (glucose or the like), or the like is used. The culture solution is not limited, but an autotrophic medium not containing an organic carbon source such as glucose as a nutrient source is preferably used. Examples of such a culture medium include a culture solution to which a nutrient salt such as a nitrogen source, a phosphorus source, or a mineral is added, such as a Cramer-Myers medium or a modified Cramer-Myers medium.

When sea water is used depending on the type of microalgae, a culture medium for marine microalgae, artificial sea water, or the like can also be used, or a commercially available product such as an IMK culture medium can also be used.

The pH of the culture solution is not particularly limited as long as the effect of the present invention is exhibited, and examples thereof include 2 to 8, 3 to 8, 4 to 8, 5 to 8, 2 to 7.5, 3 to 7.5, 4 to 7.5, and 5 to 7.5.

The culture temperature is not particularly limited as long as the effect of the present invention is exhibited, and examples thereof include 15 to 40°C, 20 to 34°C, and 23 to 28°C.

The culture period is not particularly limited as long as the effect of the present invention is exhibited, and examples thereof include 4 to 30 days, 4 to 20 days, and 5 to 15 days.

Depending on the type of microalgae, the type of light source, the presence or absence of light and darkness, aerobic conditions, anaerobic conditions, and the like can be appropriately selected.

In an embodiment, in the case of culturing marine microalgae such as Pavlova, a known method can be used as long as the effect of the present invention is exhibited. For example, culture can be performed under conditions satisfying at least one of the following:
sea water concentration: 50% seawater;
culture medium concentration: IMK medium at double strength;
culture solution amount: 800 mL;
light source: fluorescent lamp positioned at the side (100 to 150 µmol);
light/dark cycle: 12 hours light and 12 hours dark;
pH: about 7.4 at the start of each culture;
aeration: performed;
mechanical stirring: not performed;
culture period: performed with a target period of about 10 days; and
culture temperature: 25°C to 28°C.

### (Recovery of culture supernatant)

After the microalgae are cultured under the culture conditions exemplified above, the culture supernatant is recovered using a known separation means such as centrifugation. The method for recovering the culture supernatant is not particularly limited as long as the microalgae are removed or substantially removed, and the culture supernatant can be appropriately recovered. In an exemplary embodiment, the centrifugation performed to obtain the culture supernatant may be performed for 30 to 60 minutes at a centrifugal force of, for example, 1,000 to 20,000 × g, 1,500 to 20,000 × g, 1,500 to 15,000 × g, or 1,500 to 10,000 × g. At this time, the centrifugation may be performed stepwise by changing the speed or time. For example, the centrifugation may be performed at a low speed of 1,500 to 2,000 × g to separate a culture supernatant, followed by centrifugation at a high speed of 10,000 to 20,000 × g to further remove cells or cell-associated remnants, residues, and the like. The supernatant thus obtained is recovered and subjected to the next step.

### ((A) Extracellular vesicles)

In the present description, the extracellular vesicles refer to vesicles having an average diameter of usually 10 nm to 1 µm and secreted from microalgae. Such an average diameter is also referred to as an average particle diameter in the present description. Although not limited, it is preferable that the composition containing extracellular vesicles of the present invention does not contain cells themselves of microalgae. Here, the term diameter or particle diameter as used when specifying the size of extracellular vesicles refers to the maximum dimension of the extracellular vesicles, since extracellular vesicles are not necessarily spherical. The diameter or particle diameter can be measured using a microscopy technique, in particular a technique for measuring the nanoparticle size by transmission electron microscopy. The measurement can also be performed using Nanoparticle Tracking Analysis (NTA), which is based on analysis of both light scattering and Brownian motion. In the present invention, the diameter or particle diameter of extracellular vesicles is measured using NanoSight (Malvern Panalytical, NanoSight L10) for extracellular vesicles having a particle diameter of 50 nm or more, and using a transmission electron microscope for extracellular vesicles having a particle diameter of less than 50 nm.

The cumulative 10% value (D10) of particle diameter among extracellular vesicles derived from Pavlova is not particularly limited as long as the effect of the present invention is exhibited, and examples thereof include 50 to 120 nm, 60 to 110 nm, and 65 to 100 nm. Examples of the cumulative 50% value (D50) of particle diameter include 100 to 180 nm, 110 to 170 nm, and 115 to 160 nm. Examples of the cumulative 90% value (D90) of particle diameter include 180 to 300 nm, 190 to 290 nm, and 200 to 280 nm. In the present description, the cumulative 10% value (D10) of particle diameter among extracellular vesicles means a diameter at which 10% of the entire extracellular vesicles are below this value. The cumulative 50% value (D50) of particle diameter among extracellular vesicles means a diameter at which 50% of the entire extracellular vesicles are below this value, and is synonymous with the median diameter. The cumulative 90% value (D90) of particle diameter among extracellular vesicles means a diameter at which 90% of the entire extracellular vesicles are below this value.

The cumulative 10% value (D10) of particle diameter among extracellular vesicles derived from Spirulina is not particularly limited as long as the effect of the present invention is exhibited, and examples thereof include 50 to 110 nm, 60 to 100 nm, and 70 to 90 nm. Examples of the cumulative 50% value (D50) of particle diameter include 100 to 160 nm, 110 to 150 nm, and 120 to 140 nm. Examples of the cumulative 90% value (D90) of particle diameter include 190 to 250 nm, 200 to 240 nm, and 210 to 230 nm.

The cumulative 10% value (D10) of particle diameter among extracellular vesicles derived from Chlorella is not particularly limited as long as the effect of the present invention is exhibited, and examples thereof include 90 to 150 nm, 100 to 140 nm, and 110 to 130 nm. Examples of the cumulative 50% value (D50) of particle diameter include 180 to 240 nm, 190 to 230 nm, and 200 to 220 nm. Examples of the cumulative 90% value (D90) of particle diameter include 320 to 380 nm, 330 to 370 nm, and 340 to 360 nm.

Examples of the average particle count of extracellular vesicles of microalgae per mL when the composition is in a liquid state include 1.0 × 10³ or more, 1.0 × 10⁴ or more, 1.0 × 10⁵ or more, 1.0 × 10⁶ or more, 1.0 × 10⁷ or more, 1.0 × 10⁸ or more, and 1.0 × 10⁹ or more, and 1.0 × 10¹⁵ or less, 1.0 × 10¹⁴ or less, 1.0 × 10¹³ or less, 1.0 × 10¹² or less, and 1.0 × 10¹¹ or less. Examples of the average particle count of extracellular vesicles of microalgae include 1.0 × 10⁴ to 1.0 × 10¹⁵, 1.0 × 10⁴ to 1.0 × 10¹⁴, 1.0 × 10⁴ to 1.0 × 10¹³, 1.0 × 10⁴ to 1.0 × 10¹², 1.0 × 10⁴ to 1.0 × 10¹¹, 1.0 × 10⁵ to 1.0 × 10¹⁵, 1.0 × 10⁵ to 1.0 × 10¹⁴, 1.0 × 10⁵ to 1.0 × 10¹³, 1.0 × 10⁵ to 1.0 × 10¹², 1.0 × 10⁵ to 1.0 × 10¹¹, 1.0 × 10⁶ to 1.0 × 10¹⁵, 1.0 × 10⁶ to 1.0 × 10¹⁴, 1.0 × 10⁶ to 1.0 × 10¹³, 1.0 × 10⁶ to 1.0 × 10¹², 1.0 × 10⁶ to 1.0 × 10¹¹, 1.0 × 10⁷ to 1.0 × 10¹⁵, 1.0 × 10⁷ to 1.0 × 10¹⁴, 1.0 × 10⁷ to 1.0 × 10¹³, 1.0 × 10⁷ to 1.0 × 10¹², 1.0 × 10⁷ to 1.0 × 10¹¹, 1.0 × 10⁸ to 1.0 × 10¹⁵, 1.0 × 10⁸ to 1.0 × 10¹⁴, 1.0 × 10⁸ to 1.0 × 10¹³, 1.0 × 10⁸ to 1.0 × 10¹², 1.0 × 10⁸ to 1.0 × 10¹¹, 1.0 × 10⁹ to 1.0 × 10¹⁵, 1.0 × 10⁹ to 1.0 × 10¹⁴, 1.0 × 10⁹ to 1.0 × 10¹³, 1.0 × 10⁹ to 1.0 × 10¹², and 1.0 × 10⁹ to 1.0 × 10¹¹. With respect to the extracellular vesicle count, NanoSight (Malvern Panalytical, NanoSight L10) by Nanoparticle Tracking Analysis (NTA), which is based on analysis of both light scattering and Brownian motion, or the like can be used.

### (Method for obtaining extracellular vesicles)

Examples of the method for obtaining extracellular vesicles of the present invention include a method for preparing extracellular vesicles from a culture supernatant of algae.

In a certain embodiment, the extracellular vesicles of the present invention are obtained by heating a culture or a culture supernatant of microalgae.

The extracellular vesicles of the present invention can also be obtained by heating a culture or a culture supernatant of microalgae under a temperature condition of, for example, but not limited to, 40°C or higher, 50°C or higher, 60°C or higher, 70°C or higher, 80°C or higher, 90°C or higher, or 100°C or higher, and 200°C or lower, 150°C or lower, or 120°C or lower in a time condition of 30 seconds or longer, 1 minute or longer, 2 minutes or longer, 3 minutes or longer, or 4 minutes or longer, and 30 minutes or shorter, 20 minutes or shorter, or 10 minutes or shorter.

Alternatively, in another embodiment, the extracellular vesicles of the present invention can also be obtained by treating a culture supernatant of microalgae with filter filtration.

The membrane pore diameter of the filter for use in filter filtration is preferably 0.001 to 0.5 µm, more preferably 0.005 µm to 0.3 µm, still more preferably 0.01 µm to 0.25 µm.

In still another embodiment, the method for obtaining extracellular vesicles of the present invention can also include a purification step. Some specific embodiments of the purification step include purification by ultrafiltration (for example, tangential flow filtration or the like), ultracentrifugation, affinity purification using an antibody. Although not limited, among these, purification by ultrafiltration is preferable.

When purification by ultrafiltration is used, there may be an aspect in which filtration is performed from the inside to the outside of a fiber by a hollow fiber membrane, an aspect in which a spiral membrane including a filtration membrane and a support membrane is used, an aspect in which a tubular membrane on a hollow cylinder is used, or an aspect in which a flat membrane is used. Among these, it is preferable to include, but is not limited to, a step of performing filtration using a hollow fiber membrane to obtain a liquid containing extracellular vesicles in a concentrated form inside the hollow fiber membrane.

Cutoff membranes having a molecular weight cutoff of 10 kDa to 1,000 kDa, 50 kDa to 1000 kDa, and 100 kDa to 1,000 kDa are preferably used, and a cutoff membrane having a molecular weight cutoff of 100 kDa to 500 kDa is more preferably used. Cutoff membranes having a molecular weight cutoff of 10 kDa or more, 50 kDa or more, 100 kDa or more, and 150 kDa or more are preferable, and cutoff membranes having a molecular weight cutoff of 2000 kDa or less, 1500 kDa or less, 1000 kDa or less, 750 Dka or less, and 500 DKa or less are also preferable.

The extracellular vesicles of the present invention may be one of these preparation methods or a combination of two or more thereof. Although not limited to, a combination of a heat treatment step and a purification step, a combination of a purification step and a filter filtration treatment, a combination of a heat treatment step, a purification step, and a filter filtration treatment, or the like can be adopted.

The extracellular vesicles may be in the form of a purified solution of a culture supernatant, but may be a solid or a semi-solid obtained by drying the purified solution.

The extracellular vesicles can also be made into a population having high purity of the extracellular vesicles through further optional purification.

The extracellular vesicles may be in the form of a purified solution itself of a culture supernatant, but may be a solid or a semi-solid obtained by drying the purified solution.

### (Composition containing extracellular vesicles)

In one aspect of the present invention, it is possible to prepare a composition containing any of the extracellular vesicles thus obtained.

In such a composition, examples of the average particle count of extracellular vesicles of microalgae per 100 g include 1.0 × 10³ or more, 1.0 × 10⁴ or more, 1.0 × 10⁵ or more, and 1.0 × 10⁶ or more, and 1.0 × 10¹⁶ or less, 1.0 × 10¹⁵ or less, 1.0 × 10¹⁴ or less, 1.0 × 10¹³ or less, 1.0 × 10¹² or less, 1.0 × 10¹¹ or less, 1.0 × 10¹⁰ or less 1.0 × 10⁹ or less, 1.0 × 10⁸ or less, 1.0 × 10⁷ or less, and 1 × 10⁶ or less. Examples of the average particle count of extracellular vesicles of microalgae include 1.0 × 10³ to 1.0 × 10¹⁴, 1.0 × 10³ to 1.0 × 10¹³, 1.0 × 10³ to 1.0 × 10¹², 1.0 × 10³ to 1.0 × 10¹¹, 1.0 × 10³ to 1.0 × 10¹⁰, 1.0 × 10³ to 1.0 × 10⁹, 1.0 × 10³ to 1.0 × 10⁸, 1.0 × 10³ to 1.0 × 10⁷, 1.0 × 10⁴ to 1.0 × 10¹⁴, 1.0 × 10⁴ to 1.0 × 10¹³, 1.0 × 10⁴ to 1.0 × 10¹², 1.0 × 10⁴ to 1.0 × 10¹¹, 1.0 × 10⁴ to 1.0 × 10¹⁰, 1.0 × 10⁴ to 1.0 × 10⁹, 1.0 × 10⁴ to 1.0 × 10⁸, 1.0 × 10⁴ to 1.0 × 10⁷, 1.0 × 10⁵ to 1.0 × 10¹⁴, 1.0 × 10⁵ to 1.0 × 10¹³, 1.0 × 10⁵ to 1.0 × 10¹², 1.0 × 10⁵ to 1.0 × 10¹¹, 1.0 × 10⁵ to 1.0 × 10¹⁰, 1.0 × 10⁵ to 1.0 × 10⁹, 1.0 × 10⁵ to 1.0 × 10⁸, 1.0 × 10⁵ to 1.0 × 10⁷, 1.0 × 10⁶ to 1.0 × 10¹⁴, 1.0 × 10⁶ to 1.0 × 10¹³, 1.0 × 10⁶ to 1.0 × 10¹², 1.0 × 10⁶ to 1.0 × 10¹¹, 1.0 × 10⁶ to 1.0 × 10¹⁰, 1.0 × 10⁶ to 1.0 × 10⁹, 1.0 × 10⁶ to 1.0 × 10⁸, 1.0 × 10⁶ to 1.0 × 10⁷, 1.0 × 10⁷ to 1.0 × 10¹⁴, 1.0 × 10⁷ to 1.0 × 10¹³, 1.0 × 10⁷ to 1.0 × 10¹², 1.0 × 10⁷ to 1.0 × 10¹¹, 1.0 × 10⁷ to 1.0 × 10¹⁰, 1.0 × 10⁸ to 1.0 × 10¹⁴, 1.0 × 10⁸ to 1.0 × 10¹³, 1.0 × 10⁸ to 1.0 × 10¹², 1.0 × 10⁸ to 1.0 × 10¹¹, 1.0 × 10⁸ to 1.0 × 10¹⁰, 1.0 × 10⁹ to 1.0 × 10¹⁴, 1.0 × 10⁹ to 1.0 × 10¹³, 1.0 × 10⁹ to 1.0 × 10¹², 1.0 × 10⁹ to 1.0 × 10¹¹, and 1.0 × 10⁹ to 1.0 × 10¹⁰.

The average particle diameter of extracellular vesicles of microalgae is not particularly limited as long as the effect of the present invention is exhibited, and examples thereof include 10 nm or more, 20 nm or more, 30 nm or more, 40 nm or more, 50 nm or more, 60 nm or more, 70 nm or more, or 80 nm or more, and 700 nm or less, 600 nm or less, 500 nm or less, 450 nm or less, 400 nm or less, 350 nm or less, 300 nm or less, 250 nm or less, 200 nm or less, and 150 nm or less. Examples of the average particle diameter of extracellular vesicles of microalgae also include 10 to 700 nm, 10 to 600 nm, 10 to 500 nm, 10 to 400 nm, 10 to 300 nm, 10 to 250 nm, 30 to 700 nm, 30 to 600 nm, 30 to 500 nm, 30 to 400 nm, 30 to 300 nm, 30 to 250 nm, 50 to 700 nm, 50 to 600 nm, 50 to 500 nm, 50 to 400 nm, 50 to 300 nm, 50 to 250 nm, 70 to 700 nm, 70 to 600 nm, 70 to 500 nm, 70 to 400 nm, 70 to 300 nm, 70 to 250 nm, 100 to 700 nm, 100 to 600 nm, 100 to 500 nm, 100 to 400 nm, 100 to 300 nm, and 100 to 250 nm.

The content of extracellular vesicles of microalgae in the composition is appropriately adjusted depending on the type and amount of other components, the dosage form, and the like, and is not limited, but can be, for example, 0.001 mass% or more in a state of a purified solution with respect to the total amount of the composition, and may be 0.005 mass% or more, 0.01 mass% or more, 0.05 mass% or more, 0.1 mass% or more, 1 mass% or more, 5 mass% or more, 10 mass% or more, or the like. In addition, the content of extracellular vesicles of microalgae can be, for example, 50 mass% or less with respect to the total amount of the composition, and examples thereof include 40 mass% or less, 30 mass% or less, 20 mass% or less, and 15 mass% or less. Examples of the content of extracellular vesicles of microalgae with respect to the total amount of the composition include 0.001 to 50 mass%, 0.001 to 40 mass%, 0.001 to 30 mass%, 0.001 to 20 mass%, 0.001 to 15 mass%, 0.01 to 50 mass%, 0.01 to 40 mass%, 0.01 to 30 mass%, 0.01 to 20 mass%, 0.01 to 15 mass%, 0.1 to 50 mass%, 0.1 to 40 mass%, 0.1 to 30 mass%, 0.1 to 20 mass%, 0.1 to 15 mass%, 1 to 50 mass%, 1 to 40 mass%, 1 to 30 mass%, 1 to 20 mass%, and 1 to 15 mass%.

The content of extracellular vesicles of microalgae is appropriately adjusted depending on the type and amount of other components, the dosage form, and the like, and is not limited, but can be, for example, 0.00001 µg/mL or more, 0.0001 µg/mL or more, 0.001 µg/mL or more, 0.01 µg/mL or more, 0.1 µg/mL or more, or 1 µg/mL or more in terms of protein amount with respect to the total amount of the composition. In addition, examples thereof include 100 µg/mL or less, 10 µg/mL or less, 1 µg/mL, 0.1 µg/mL or less, and 0.01 µg/mL or less.

### ((B-1) One type or two or more types selected from group consisting of primary alcohol and polyhydric alcohol)

The component (B-1) of the present invention is at least one type selected from the group consisting of a primary alcohol and a polyhydric alcohol.

The primary alcohol that can be used in the present invention is not limited as long as the primary alcohol is used in the pharmaceutical or cosmetic field, and optionally the food field. From the viewpoint of remarkably exhibiting the effect of the present invention, the primary alcohol preferably has 1 to 3 carbon atoms. Examples thereof include methanol, ethanol, n-propanol, and isopropanol, and the primary alcohol used in the present invention preferably contains at least ethanol, and is more preferably ethanol. In the present invention, the primary alcohol may be used alone or in combination of two or more thereof.

The polyhydric alcohol that can be used in the present invention is not limited as long as the polyhydric alcohol is used in the pharmaceutical or cosmetic field, and optionally the food field. In the present description, the "polyhydric alcohol" refers to an alcohol having two or more hydroxy (-OH) groups in one molecule. Although not limited, as the polyhydric alcohol, dihydric to hexahydric alcohols are preferably used, and dihydric to pentahydric alcohols are more preferable, and dihydric to trihydric alcohols are still more preferable from the viewpoint of exhibiting the effect of the present invention. Here, examples of the dihydric alcohol include dipropylene glycol, 1,3-butylene glycol, 1,3-propanediol, 3-methyl-1,3-butanediol, propylene glycol, pentanediol, hexanediol, octanediol, and polyethylene glycol; examples of the trihydric alcohol include glycerin; examples of the tetrahydric alcohol include pentaerythritol; examples of the pentahydric alcohol include diglycerin and xylitol; and examples of the hexahydric alcohol include sorbitol and inositol. The polyhydric alcohol may be used alone or in combination of two or more thereof. As the polyhydric alcohol, one or more types selected from the group consisting of glycerin, dipropylene glycol, 1,3-butylene glycol, 1,3-propanediol, pentanediol, and polyethylene glycol are preferable, and glycerin, 1,3-butylene glycol, 1,3-propanediol, and polyethylene glycol are particularly preferable.

Examples of the polyethylene glycol include macrogol 200 (liquid), macrogol 300 (liquid), and macrogol 1540 (paste) in the Japanese Pharmaceutical Excipients, macrogol 400 (liquid), macrogol 1500 (paste), and macrogol 4000 (solid) in the Japanese Pharmacopoeia, polyethylene glycol 200 (liquid), polyethylene glycol 300 (liquid), polyethylene glycol 400 (liquid), polyethylene glycol 600 (liquid), polyethylene glycol 1000 (wax), polyethylene glycol 1500 (paste), polyethylene glycol 1540 (wax), polyethylene glycol 2000 (wax), and polyethylene glycol 4000 (solid) in the Japanese Standards of Quasi-Drug Ingredients 2006.

As described above, the properties of polyethylene glycol vary depending on the molecular weight. In the present invention, it is preferable to use polyethylene glycol in the form of a liquid, paste, or wax, more preferably in the form of a liquid or paste, and still more preferably in the form of a liquid.

The total content of the component (B-1) with respect to the total amount of the composition of the present invention is preferably 1 to 60 mass%, more preferably 3 to 50 mass%, and still more preferably about 5 to 40 mass%.

The content of the primary alcohol with respect to the total amount of the composition of the present invention is preferably 0.01 to 30 mass%, more preferably 1 to 25 mass%, and still more preferably about 3 to 20 mass%.

The total content of the polyhydric alcohol with respect to the total amount of the composition of the present invention is preferably 0.5 to 50 mass%, more preferably 2 to 40 mass%, and still more preferably about 3 to 30 mass%.

In the composition of the present invention, the count, with respect to 1 mass% of the primary alcohol, of the component (A) is preferably 1 × 10 to 1 × 10¹⁰, more preferably 1 × 10² to 1 × 10⁹, still more preferably 1 × 10³ to 1 × 10⁸, and even more preferably 1 × 10⁴ to 1 × 10⁷.

In the composition of the present invention, the count, with respect to 1 mass% of the polyhydric alcohol, of the component (A) is preferably 1 × 10 to 1 × 10¹⁰, more preferably 1 × 10² to 1 × 10⁹, still more preferably 1 × 10³ to 1 × 10⁸, and even more preferably 1 × 10⁴ to 1 × 10⁷.

### ((B-2) One type or two or more types selected from group consisting of organic acid and salt thereof)

The component (B-2) of the present invention is an organic acid or a salt thereof. In the present invention, the organic acid is an acid of an organic compound having an organic group and having a molecular weight of 1000 or less, preferably 700 or less, more preferably 500 or less, still more preferably 350 or less, and particularly preferably 300 or less. The organic acid is preferably a water-soluble organic acid. The organic acid may be a carboxylic acid having a carboxyl group, a sulfonic acid having a sulfo group, or a phosphoric acid ester having a phosphoric acid group. More specific examples of the organic acid include, but are not limited to, ascorbic acid, tranexamic acid, citric acid, salicylic acid, lactic acid, tartaric acid, malic acid, succinic acid, oxalic acid, gluconic acid, fumaric acid, aspartic acid, pyrrolidone carboxylic acid, ε-aminocaproic acid, glutamic acid, aminoethyl sulfonic acid, ellagic acid, kojic acid, glycyrrhizic acid, glycyrrhetinic acid, phytic acid, ferulic acid, glycolic acid and azelaic acid. Among these, at least one type selected from the group consisting of ascorbic acid, tranexamic acid, citric acid, salicylic acid, lactic acid, tartaric acid, malic acid, succinic acid, gluconic acid, aspartic acid, pyrrolidone carboxylic acid, ε-aminocaproic acid, glutamic acid, aminoethyl sulfonic acid, ellagic acid, kojic acid, glycyrrhizic acid, glycyrrhetinic acid, phytic acid, ferulic acid, glycolic acid, and azelaic acid is exemplified. Among these, at least one type selected from the group consisting of ascorbic acid, tranexamic acid, citric acid, salicylic acid, lactic acid, tartaric acid, malic acid, succinic acid, gluconic acid, pyrrolidone carboxylic acid, ε-aminocaproic acid, glutamic acid, kojic acid, glycyrrhizic acid, glycyrrhetinic acid, phytic acid, ferulic acid, glycolic acid, and azelaic acid is preferable, at least one type selected from the group consisting of ascorbic acid, tranexamic acid, citric acid, salicylic acid, lactic acid, succinic acid, tartaric acid, gluconic acid, kojic acid, phytic acid, glycolic acid, and glycyrrhizic acid is more preferable, and at least one type selected from citric acid and succinic acid is still more preferable.

In the present invention, the organic acid salt refers to a salt of such an organic acid. A salt forming an organic acid salt is a pharmaceutically acceptable salt. Examples thereof include, but are not limited to, salts with organic bases (for example, salts with tertiary amines such as trimethylamine salts, triethylamine salts, monoethanolamine salts, triethanolamine salts, and pyridine salts; basic ammonium salts such as arginine or lysine, and the like), or salts with inorganic bases (for example, inorganic acid salts such as hydrochlorides, sulfates, and phosphates: ammonium salts; alkali metal salts such as sodium salts and potassium salts; alkaline earth metal salts such as calcium salts and magnesium salts; zinc salts, aluminum salts, and the like). Among these, preferable organic acid salts are triethanolamine salts, monoammonium salts, sodium salts, potassium salts, dipotassium salts, magnesium salts, or zinc salts, and particularly preferable organic acid salts are sodium salts, dipotassium salts, or magnesium salts.

In the present invention, preferable examples of the organic acid salt include sodium ascorbate, tranexamate hydrochloride, calcium tranexamate, sodium salicylate, calcium salicylate, magnesium salicylate, potassium salicylate, sodium lactate, sodium tartrate, sodium citrate, disodium citrate, trisodium citrate, potassium citrate, sodium succinate, disodium succinate, sodium oxalate, calcium gluconate, zinc gluconate, sodium pyrrolidone carboxylate, zinc pyrrolidone carboxylate, sodium glutamate, dipotassium glycyrrhizinate, monoammonium glycyrrhizinate, sodium phytate, sodium glycolate, and ammonium glycolate. Among these, at least one type selected from the group consisting of sodium ascorbate, tranexamate hydrochloride, calcium tranexamate, sodium salicylate, sodium lactate, sodium tartrate, sodium citrate, disodium citrate, trisodium citrate, sodium succinate, disodium succinate, calcium gluconate, zinc gluconate, zinc pyrrolidone carboxylate, sodium pyrrolidone carboxylate, sodium glutamate, dipotassium glycyrrhizinate, monoammonium glycyrrhizinate, sodium phytate, sodium glycolate, ammonium glycolate, sodium succinate, and disodium succinate is preferable, and at least one type selected from the group consisting of sodium ascorbate, sodium salicylate, sodium lactate, sodium citrate, disodium citrate, trisodium citrate, zinc gluconate, sodium pyrrolidone carboxylate, sodium glutamate, sodium succinate, and disodium succinate is more preferable.

The total content of the organic acid or the salt thereof with respect to the total amount of the composition of the present invention is preferably 0.0001 to 30 mass%, more preferably 0.001 to 10 mass%, and still more preferably about 0.01 to 5 mass%.

In the composition of the present invention, the count, with respect to 1 mass% of the organic acid or the salt thereof, of the component (A) is preferably 1 × 10 to 1 × 10¹⁶, more preferably 1 × 10² to 1 × 10¹⁰, still more preferably 1 × 10³ to 1 × 10⁸, and even more preferably 1 × 10⁴ to 1 × 10⁷.

### ((B-3) Chelating agent)

The component (B-3) of the present invention is a chelating agent. Examples of the chelating agent used in the present invention include ethylenediaminetetraacetic acid (edetic acid), ethylenediaminetetraacetates (a sodium salt (sodium edetate: Japanese Pharmacopoeia, EDTA-2Na, and the like), a potassium salt, and the like), phytic acid, gluconic acid, polyphosphoric acid, and metaphosphoric acid. Among these, disodium edetate is preferable.

The total content of the chelating agent with respect to the total amount of the composition of the present invention is preferably 0.0001 to 2 mass%, more preferably 0.001 to 1 mass%, and still more preferably about 0.01 to 0.5 mass%.

In the composition of the present invention, the count, with respect to 1 mass% of the chelating agent, of the component (A) is preferably 1 × 10 to 1 × 10¹⁶, more preferably 1 × 10² to 1 × 10¹⁰, still more preferably 1 × 10³ to 1 × 10⁸, and even more preferably 1 × 10⁴ to 1 × 10⁷.

### ((C) Water)

The composition of the present invention can also contain water. The total content of water with respect to the total amount of the composition of the present invention is preferably 1 mass% or more, 10 mass% or more, 20 mass% or more, 30 mass% or more, 40 mass% or more, 50 mass% or more, 60 mass% or more, 70 mass% or more, 80 mass% or more, or 90 mass% or more, and is preferably 99 mass% or less, 90 mass% or less, 80 mass% or less, 70 mass% or less, 60 mass% or less, 50 mass% or less, or 40 mass% or less.

In the composition of the present invention, the count, with respect to 1 mass% of water, of the component (A) is preferably 1 × 10 to 1 × 10¹⁶, more preferably 1 × 10² to 1 × 10¹⁴, still more preferably 1 × 10³ to 1 × 10¹², and even more preferably 1 × 10⁴ to 1 × 10¹¹. In a certain embodiment, the count, with respect to 1 mass% of water, of the component (A) is preferably 1 × 10 to 1 × 10¹⁰, more preferably 1 × 10² to 1 × 10⁹, still more preferably 1 × 10³ to 1 × 10⁸, and even more preferably 1 × 10⁴ to 1 × 10⁷.

In the composition of the present invention, the pH may be 2.5 or more, 3.5 or more, 4.0 or more, 5.0 or more, or 6.0 or more, and may be 14 or less, 13 or less, 12 or less, 11 or less, 10 or less, 9 or less, or 8 or less. The pH of the composition may be pH 2.5 to 12, pH 3.5 to 10, or pH 5 to 8.

### (Nonionic surfactant)

The composition of the present invention may contain a nonionic surfactant in addition to the components (A) and (B) as long as the effect of the present invention is not hindered. The nonionic surfactant that can be used in the present invention is not limited, but is preferably a nonionic surfactant having an HLB of less than 15, more preferably a surfactant having an HLB of 14.5 or less, and still more preferably a surfactant having an HLB of 14 or less. The HLB of the nonionic surfactant may be 6 to 14.5, preferably 8 to 14.5, and more preferably 10 to 14.5.

The hydrophilic-lipophilic balance (HLB) value in the present invention refers to a value calculated by Kawakami formula (Formula 1 below), which utilizes the hydrophilic group molecular weight (Mw) and the lipophilic group molecular weight (MO) of the surfactant molecule.$HLB value = 7 + 11.7 logMw / Mo$ (Formula 1)

Examples of the nonionic surfactant include polyglycerin fatty acid esters such as hexaglyceryl monolaurate (HLB value: 14.5), hexaglyceryl monomyristate (HLB value: 11), hexaglyceryl monostearate (HLB value: 9.0), hexaglyceryl monooleate (HLB value: 9.0), decaglyceryl monomyristate (HLB value: 14.0), decaglyceryl monostearate (HLB value: 12.0), decaglyceryl monoisostearate (HLB value: 12.0), decaglyceryl monooleate (HLB value: 12.0), decaglyceryl distearate (HLB value: 9.5), and decaglyceryl diisostearate (HLB value: 10.0);
polyoxyethylene glycerin fatty acid esters such as polyoxyethylene 5 mol adduct (referred to as "POE(5)", hereinafter the same shall apply) glyceryl monostearate (HLB value: 9.5), POE(15) glyceryl monostearate (HLB value: 13.5), POE(5) glyceryl monooleate (HLB value: 9.5), and POE(15) glyceryl monooleate (HLB value: 14.5); polyoxyethylene sorbitan fatty acid esters such as POE(20) sorbitan monostearate (HLB value: 14.9), POE(6) sorbitan monostearate (HLB value: 9.5), POE(20) sorbitan tristearate (HLB value: 10.5), POE(20) sorbitan monoisostearate (HLB value: 15.0), POE(6) sorbitan monooleate (HLB value: 10.0), and POE(20) sorbitan trioleate (HLB value: 11.0);
polyoxyethylene sorbitol fatty acid esters such as POE(60) sorbitol tetrastearate (HLB value: 13.0), POE(30) sorbitol tetraoleate (HLB value: 11.5), POE(40) sorbitol tetraoleate (HLB value: 12.5), and POE(60) sorbitol tetraoleate (HLB value: 14.0);
polyoxyethylene lanolins/lanolin alcohols/beeswax derivatives such as POE(10) lanolin (HLB value: 12.0), POE(20) lanolin (HLB value: 13.0), POE(5) lanolin alcohol (HLB value: 12.5), and POE(20) sorbitol beeswax (HLB value: 9.5); polyoxyethylene castor oils/hydrogenated castor oils such as POE(20) castor oil (HLB value: 10.5), POE(40) castor oil (HLB value: 12.5), POE(50) castor oil (HLB value: 14.0), POE(60) castor oil (HLB value: 14.0), POE(20) hydrogenated castor oil (HLB value: 10.5), POE(30) hydrogenated castor oil (HLB value: 11.0), POE(40) hydrogenated castor oil (HLB value: 13.5), and POE(60) hydrogenated castor oil (HLB value: 14.0);
polyoxyethylene sterols/hydrogenated sterols such as POE(5) phytosterol (HLB value: 9.5), POE(10) phytosterol (HLB value: 12.5), and POE(25) phytostanol (HLB value: 14.5);
polyoxyethylene alkyl ethers such as POE(2) lauryl ether (HLB value: 9.5), POE(4.2) lauryl ether (HLB value: 11.5), POE(9) lauryl ether (HLB value: 14.5), POE(5.5) cetyl ether (HLB value: 10.5), POE(7) cetyl ether (HLB value: 11.5), POE(10) cetyl ether (HLB value: 13.5), POE(4) stearyl ether (HLB value: 9.0), POE(7) oleyl ether (HLB value: 10.5), POE(10) oleyl ether (HLB value: 14.5), POE(10) behenyl ether (HLB value: 10.0), POE(2) (C12-15) alkyl ether (HLB value: 9.0), POE(4) (C12-15) alkyl ether (HLB value: 10.5), POE(5) secondary alkyl ether (HLB value: 10.5), POE(7) secondary alkyl ether (HLB value: 12.0), POE(9) alkyl ether (HLB value: 13.5), and POE(12) alkyl ether (HLB value: 14.5);
polyoxyethylene polyoxypropylene alkyl ethers such as polyoxypropylene 1 mol adduct (referred to as "POP(1)", hereinafter the same shall apply) POP(4) cetyl ether (HLB value: 9.5), POE(10)POP(4) cetyl ether (HLB value: 10.5), POE(20)POP(8) cetyl ether (HLB value: 12.5), POE(20)POP(6) decyltetradecyl ether (HLB value: 11.0), and POE(30)POP(6) decyltetradecyl ether (HLB value: 12.0);
polyethylene glycol fatty acid esters such as polyethylene glycol 10 mol adduct (referred to as "PEG(10)", hereinafter the same shall apply) monolaurate (HLB value: 12.5), PEG(10) monostearate (HLB value: 11.0), PEG(10) monooleate (HLB value: 11.0), and PEG diisostearate (HLB value: 9.5); and polyoxyethylene glyceryl isostearates such as PEG(8) glyceryl isostearate (HLB value: 10.0), PEG(10) glyceryl isostearate (HLB value: 10.0), PEG(15) glyceryl isostearate (HLB value: 12.0), PEG(20) glyceryl isostearate (HLB value: 13.0), PEG(25) glyceryl isostearate (HLB value: 14.0), and PEG glyceryl (30) isostearate.

The total content of the nonionic surfactant having an HLB of less than 15 with respect to the total amount of the composition of the present invention is preferably 0.01 to 5 mass%, more preferably 0.1 to 4 mass%, and still more preferably about 0.5 to 3 mass%.

### (Additives)

The composition of the present invention may contain various additives other than the above components as long as the effect of the present invention is not impaired. Examples of the additive include an antioxidant, a thickener, a pH adjusting agent, an inorganic salt, a stabilizer, an ultraviolet absorber or an ultraviolet scattering agent, an irritation reducer, a colorant, and a fragrance. One type or two or more types of additives can be used.

Here, the inorganic salt is preferably sodium chloride, disodium hydrogen phosphate, potassium chloride, or potassium dihydrogen phosphate.

### [Application]

In an embodiment, the composition containing extracellular vesicles of the present invention is suitably used as an internal or external preparation.

The composition of the present invention can be used by being added to or mixed with a pharmaceutical, a quasi-drug, a cosmetic, a food, a beverage, a feed, or a pet food. Alternatively, the composition of the present invention can be used as it is as a pharmaceutical, a quasi-drug, a cosmetic, a food, a beverage, a feed, a pet food, or the like. Furthermore, the composition may be a raw material for preparing such a product or a so-called premix product.

When used in so-called external preparations such as pharmaceuticals, quasi-drugs, and cosmetics, the composition of the present invention is preferably exemplified by skin-care cosmetics (for example, lotions, creams, facial creams, facial lotions, milky lotions, facial packs, liquid facial cleansers, soaps, and beauty serums), makeup cosmetics (for example, eyeliners and eyebrow cosmetics), and scalp and hair cosmetics (for example, scalp lotions, scalp creams, shampoos, hair conditioners, hair treatments, hair essences, hair mists, and hair tonics). More preferably, the composition is in the form of a lotion, cream, facial cream, facial lotion, milky lotion, facial pack, facial cleanser, soap, or beauty serum, and particularly preferably a lotion, facial lotion, or beauty serum, but not limited thereto.

Thus, when used in so-called external preparations such as pharmaceuticals, quasi-drugs, and cosmetics, the dosage form of the composition of the present invention is not particularly limited, and examples thereof include liquids, suspensions, emulsions, creams, gels, liniments, lotions, ointments, and aerosols. Among these, liquids, suspensions, emulsions, lotions, and aerosols are preferable, and liquids, lotions, and aerosols are more preferable. In addition, a premix product may be, for example, a liquid obtained by mixing extracellular vesicles, water, a glycol or a preservative, an excipient, a dissolution aid, a pH adjusting agent, or the like.

When used in so-called external preparations such as pharmaceuticals, quasi-drugs, and cosmetics, the adult daily dose of extracellular vesicles of microalgae can be appropriately determined depending on the individual condition, body weight, sex, age, activity of the material, route of intake or administration, schedule of intake or administration, formulation form, or other factors. Examples of the adult daily dose of extracellular vesicles of microalgae include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more. Examples of the adult daily dose of extracellular vesicles of microalgae include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily dose of extracellular vesicles of microalgae include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 ug. In addition, the dose of extracellular vesicles of microalgae varies depending on various factors such as the condition and age of the subject, but when an adult is used as a reference, generally, 1 µg/kg to 200 mg/kg of the composition, or 50 µg/kg to 50 mg/kg in another aspect may be administered 1 to 3 times a day, and the dose does not limit the range of the present specification by any method.

When used in so-called external preparations such as pharmaceuticals, quasi-drugs, and cosmetics, the adult daily dose of extracellular vesicles of microalgae can be appropriately determined depending on the individual condition, body weight, sex, age, activity of the material, route of intake or administration, schedule of intake or administration, formulation form, or other factors. Examples of the adult daily dose of extracellular vesicles of microalgae include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more in terms of protein amount. Examples of the adult daily dose of extracellular vesicles of microalgae include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily dose of extracellular vesicles of microalgae include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg. In addition, the dose of extracellular vesicles of microalgae varies depending on various factors such as the condition and age of the subject, but when an adult is used as a reference, 1 µg/kg to 200 mg/kg of the composition, or 50 µg/kg to 50 mg/kg in another aspect may be administered 1 to 3 times a day, and the dose does not limit the range of the present specification by any method.

In another embodiment, the adult daily dose of extracellular vesicles of microalgae is, for example, 1 × 10 or more, 1 × 10² or more, 1 × 10³ or more, or 1 × 10⁴ or more in terms of extracellular vesicle count. Examples of the adult daily dose of extracellular vesicles of microalgae include 1 × 10 to 1 × 10¹⁰, 1 × 10 to 1 × 10⁹, 1 × 10 to 1 × 10⁸, 1 × 10 to 1 × 10⁷, 1 × 10² to 1 × 10¹⁰, 1 × 10² to 1 × 10⁹, 1 × 10² to 1 × 10⁸, 1 × 10² to 1 × 10⁷, 1 × 10³ to 1 × 10¹⁰, 1 × 10³ to 1 × 10⁹, 1 × 10³ to 1 × 10⁸, 1 × 10³ to 1 × 10⁷, 1 × 10⁴ to 1 × 10¹⁰, 1 × 10⁴ to 1 × 10⁹, 1 × 10⁴ to 1 × 10⁸, and 1 × 10⁴ to 1 × 10⁷ in terms of extracellular vesicle count.

The composition of the present invention may be taken or administered in 1 to several portions per day, usually 1 to 6 times per day, 1 to 3 times per day, 1 to 2 times per day, or at any period and interval, but preferably twice per day.

When the composition of the present invention is used by being added to or mixed with a food, a beverage, a feed, or a pet food, the composition of the present invention can also be used as a food or beverage, that is, as a health food, a food labeled with functionality, a food for patients, and a food for specified health uses. The composition of the present invention can also be used as a so-called doctor's supplement recommended or presented by a doctor in a hospital and/or a doctor's office, or internal medicine, orthopedic surgery, animal hospital, or the like.

Specifically, the health food, the food labeled with functionality, the food for patients, and the food for specified health uses can be used in various formulation forms such as solid preparations (tablets, orally disintegrating tablets, granules, fine granules, powders, capsules, chewable tablets, candies, and the like), liquid preparations (syrups and suspensions), and liquid foods. A food in such a formulation form can be produced in the same manner as known pharmaceutical preparations, and can be produced by mixing an active ingredient with a carrier acceptable as a food, for example, an appropriate excipient or the like, and then using conventional means. The formulation form is not limited, but is preferably an orally disintegrating tablet, a chewable tablet, a candy, a granule, a powder, or a liquid from the viewpoint of remarkably exhibiting the effect of the present invention.

In addition, the composition of the present invention can be produced as liquid beverages such as soups, juices, fruit juice beverages, milk, milk beverages, whey beverages, lactic acid bacteria beverages, tea beverages, alcoholic beverages, coffee beverages, carbonated beverages, soft drinks, water beverages, cocoa beverages, jelly-like beverages, sports beverages, and dietetic beverages, semi-solid foods such as puddings and yogurts, noodles such as pasta, ramen, Japanese noodles, and buckwheat noodles, confectionery, spreads, and the like.

The composition of the present invention can be applied in the form of oral use, internal use, and the like. When used as a pharmaceutical composition, the composition of the present invention may be used therapeutically or non-therapeutically.

The adult daily oral intake amount or dose of extracellular vesicles of microalgae can be appropriately determined depending on the individual condition, body weight, sex, age, activity of the material, route of intake or administration, schedule of intake or administration, formulation form, or other factors. Examples of the adult daily oral intake amount or dose of extracellular vesicles of microalgae include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more. Examples of the adult daily oral intake amount or dose of extracellular vesicles of microalgae include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily oral intake amount or dose of extracellular vesicles of microalgae include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg.

In another embodiment, the adult daily oral intake amount or dose of extracellular vesicles of microalgae is, for example, 1 × 10 or more, 1 × 10² or more, 1 × 10³ or more, or 1 × 10⁴ or more in terms of extracellular vesicle count. In addition, the adult daily oral intake amount or dose of extracellular vesicles of microalgae is, for example, 1 × 10⁹ or less, 1 × 10⁸ or less, 1 × 10⁷ or less, 1 × 10⁶ or less, or 1 × 10⁵ or less in terms of extracellular vesicle count. Examples of the adult daily oral intake amount or dose of extracellular vesicles of microalgae include 1 × 10 to 1 × 10⁹, 1 × 10 to 1 × 10⁸, 1 × 10 to 1 × 10⁷, 1 × 10 to 1 × 10⁶, 1 × 10² to 1 × 10⁹, 1 × 10² to 1 × 10⁸, 1 × 10² to 1 × 10⁷, 1 × 10² to 1 × 10⁶, 1 × 10³ to 1 × 10⁹, 1 × 10³ to 1 × 10⁸, 1 × 10³ to 1 × 10⁷, 1 × 10³ to 1 × 10⁶, 1 × 10⁴ to 1 × 10⁹, 1 × 10⁴ to 1 × 10⁸, 1 × 10⁴ to 1 × 10⁷, and 1 × 10⁴ to 1 × 10⁶ in terms of extracellular vesicle count.

The adult daily oral intake amount or dose may be divided depending on the dosage form, for example, into 1 to 6 capsules, 1 to 4 capsules, 1 to 3 capsules, or 1 to 2 capsules, in the case of capsules.

The composition of the present invention may be taken or administered in 1 to several portions per day, usually 1 to 6 times per day, 1 to 3 times per day, 1 to 2 times per day, or at any period and interval, but preferably once per day.

When the present invention is used as a feed, a pet food, or the like, the target organism is not particularly limited, but is preferably a mammal, a reptile, an amphibian, a bird, or a fish, and more preferably a mammal other than humans.

When the present invention is used as a feed, a pet food, or the like, the composition may be fed several times a day by adding the composition to a staple food or the like, or may be given as a snack as needed.

### [Method for improving stability]

The present invention relates to a method for improving the stability of microalgae-derived extracellular vesicles (A) by allowing one type or two or more types selected from a group consisting of (B-1) to (B-3) to coexist with the microalgae-derived extracellular vesicles:
(B-1) one type or two or more types selected from a group consisting of a primary alcohol and a polyhydric alcohol;
(B-2) one type or two or more types selected from a group consisting of an organic acid and a salt thereof; and
(B-3) a chelating agent.

The present invention also relates to a composition containing microalgae-derived extracellular vesicles (A) and having improved stability.

The present invention also relates to a composition that contains microalgae-derived extracellular vesicles (A) and one type or two or more types selected from a group consisting of (B-1) to (B-3), and that has improved stability of the microalgae-derived extracellular vesicles:
(B-1) one type or two or more types selected from a group consisting of a primary alcohol and a polyhydric alcohol;
(B-2) one type or two or more types selected from a group consisting of an organic acid and a salt thereof; and
(B-3) a chelating agent.

Here, the improvement in stability refers to, for example, little variation in the particle count and/or size of extracellular vesicles before and after storage of the composition containing extracellular vesicles.

Extracellular vesicles are expected to have various physiologically active functions, and the maintenance of the count and physical properties thereof leads to the maintenance of the properties of the composition containing extracellular vesicles.

The present invention includes the following aspects.
[1] A composition containing microalgae-derived extracellular vesicles (A).
[2] A composition containing:
   microalgae-derived extracellular vesicles (A); and
   one type or two or more types selected from a group consisting of (B-1) to (B-3):
      (B-1) one type or two or more types selected from a group consisting of a primary alcohol and a polyhydric alcohol;
      (B-2) one type or two or more types selected from a group consisting of an organic acid and a salt thereof; and
      (B-3) a chelating agent.
[3] The composition according to [2], wherein the composition contains the component (B-1), and contains, as the component (B-1), one type or two or more types selected from a group consisting of a primary alcohol having 1 to 3 carbon atoms, a dihydric to trihydric polyhydric alcohol, and polyethylene glycol.
[4] The composition according to [3], wherein the primary alcohol is one or more types selected from a group consisting of methanol, ethanol, n-propanol, and isopropanol.
[5] The composition according to [3], wherein the dihydric to trihydric polyhydric alcohol is one or more types selected from a group consisting of dipropylene glycol, 1,3-butylene glycol, 1,3-propanediol, 3-methyl-1,3-butanediol, propylene glycol, pentanediol, hexanediol, octanediol or polyethylene glycol, and glycerin.
[6] The composition according to any one of [2] to [5], wherein the composition contains the component (B-2), and contains, as the component (B-2), one type or two or more types selected from a group consisting of an organic acid having a molecular weight of 300 or less and a salt thereof.
[7] The composition according to [6], wherein the organic acid having a molecular weight of 300 or less and a salt thereof are one or more types selected from a group consisting of ascorbic acid, tranexamic acid, citric acid, salicylic acid, lactic acid, succinic acid, tartaric acid, gluconic acid, kojic acid, phytic acid, glycolic acid, and glycyrrhizic acid.
[8] The composition according to any one of [2] to [7], wherein the composition contains the component (B-3) and contains, as the component (B-3), an ethylenediaminetetraacetate.
[9] A composition containing microalgae-derived extracellular vesicles (A) (excluding an anti-aging composition).
[10] The composition according to any one of [1] to [9], further containing water.
[11] The composition according to any one of [1] to [10], wherein the microalgae are microalgae belonging to the genus Pavlova, Euglena, Spirulina, or Chlorella.
[12] The composition according to any one of [1] to [11], which is for internal use or external use.
[13] An external composition containing:
   (A) extracellular vesicles derived from Pavlova; and
   (B-1) 1,3-butylene glycol.
[14] An external composition containing:
   (A) extracellular vesicles derived from Pavlova; and
   (B-1) 1,3-propanediol.
[15] An external composition containing:
   (A) extracellular vesicles derived from Pavlova; and
   (B-1) dipropylene glycol.
[16] An external composition containing:
   (A) extracellular vesicles derived from Pavlova; and
   (B-1) glycerin.
[17] An external composition containing:
   (A) extracellular vesicles derived from Pavlova; and
   (B-1) 1,2-pentanediol.
[18] An external composition containing:
   (A) extracellular vesicles derived from Pavlova; and
   (B-1) polyethylene glycol.
[19] An external composition containing:
   (A) extracellular vesicles derived from Pavlova; and
   (B-1) ethanol.
[20] An external composition containing:
   (A) extracellular vesicles derived from Pavlova; and
   (B-2) citric acid and/or a salt thereof.
[21] An external composition containing:
   (A) extracellular vesicles derived from Pavlova; and
   (B-3) an ethylenediaminetetraacetate.
[22] A method for improving stability of microalgae-derived extracellular vesicles (A) by allowing one type or two or more types selected from a group consisting of (B-1) to (B-3) to coexist with the microalgae-derived extracellular vesicles:
   (B-1) one type or two or more types selected from a group consisting of a primary alcohol and a polyhydric alcohol;
   (B-2) one type or two or more types selected from a group consisting of an organic acid and a salt thereof; and
   (B-3) a chelating agent.
[23] A method for improving stability of microalgae-derived extracellular vesicles (A) in a composition by allowing the microalgae-derived extracellular vesicle; and one type or two or more types selected from a group consisting of one type or two or more types selected from a group consisting of a primary alcohol having 1 to 3 carbon atoms, a dihydric to trihydric polyhydric alcohol, and polyethylene glycol (B-1) to coexist.
[24] A method for improving stability of microalgae-derived extracellular vesicles (A) in an external composition or an internal composition by allowing one type or two or more types selected from a group consisting of (B-1) to (B-3) to coexist with the microalgae-derived extracellular vesicles:
   (B-1) one type or two or more types selected from a group consisting of a primary alcohol having 1 to 3 carbon atoms, a dihydric to trihydric polyhydric alcohol, and polyethylene glycol;
   (B-2) one type or two or more types selected from a group consisting of an organic acid having a molecular weight of 300 or less and a salt thereof; and
   (B-3) an ethylenediaminetetraacetate.
[25] The method according to any one of [22] to [24], wherein the composition further contains water.
[26] The method according to any one of [22] to [25], wherein the microalgae are microalgae belonging to the genus Pavlova, Euglena, Spirulina, or Chlorella.

### EXAMPLES

Next, a specific description is made of the present invention with reference to examples, but the present invention is not limited to the following examples.

### (Preparation Examples)

Using a commercially available medium for algae, Pavlova sp., Arthrospira platensis, or Chlorella vulgaris was cultured by a conventional method to yield a culture supernatant liquid. Each culture supernatant was heat-treated at 105°C for 4 minutes. After heating, 3000 mL of the supernatant obtained through centrifugation at 3000 × g for 60 minutes at room temperature was filtered through a 0.2 µm filter, and then, the filtrate was concentrated to a volume of 150 mL or less by a tangential flow method using a 300 kDa hollow fiber membrane filter (MiniKros sampler S02-E300-05-N, manufactured by Repligen). Thereafter, concentration and washing were performed using 10,000 mL of PBS, and finally, 100 mL was recovered with PBS. Thereafter, each concentrated sample was subjected to filter sterilization using a 0.22 µm filter, and the particle count and the like were measured using NanoSight.

Each of the samples was appropriately diluted with PBS(-), and a count distribution graph (horizontal axis: particle diameter, vertical axis: particle count concentration) was created according to the attached software using a nanoparticle analysis system NanoSight LM10 (manufactured by Malvern Panalytical). The particle diameter and the particle size were measured three times, and the average values thereof were taken as the average particle diameter and the average particle size of each sample.

The following samples were obtained as described above.
· Ultrafiltered Pavlova sp. culture supernatant sample (protein concentration 574 µg/mL)
· Ultrafiltered Arthrospira platensis culture supernatant sample (protein concentration 29.6 µg/mL)
· Ultrafiltered Chlorella vulgaris culture supernatant sample (protein concentration 10.4 µg/mL)

In the ultrafiltered Pavlova sp. culture supernatant sample, the particle count of Pavlova sp.-derived extracellular vesicles was 1.79 × 10¹²/mL, the average particle diameter was 146 nm, the cumulative 10% value of particle diameter (D10) was 75 nm, the cumulative 50% value of particle diameter (D50) was 135.3 nm, and the cumulative 90% value of particle diameter (D90) was 229.3 nm.

In the ultrafiltered Arthrospira platensis culture supernatant sample, the particle count of Arthrospira platensis-derived extracellular vesicles was 2.88 × 10¹¹/mL, the average particle diameter was 138.7 nm, the cumulative 10% value of particle diameter (D10) was 72.2 nm, the cumulative 50% value of particle diameter (D50) was 125.9 nm, and the cumulative 90% value of particle diameter (D90) was 217.8 nm.

In the ultrafiltered Chlorella vulgaris culture supernatant sample, the particle count of Chlorella vulgaris-derived extracellular vesicles was 8.18 × 10⁹/mL, the average particle diameter was 227.7 nm, the cumulative 10% value of particle diameter (D10) was 121.4 nm, the cumulative 50% value of particle diameter (D50) was 215.8 nm, and the cumulative 90% value of particle diameter (D90) was 348.5 nm.

### (Examples and Comparative Examples)

Compositions having the formulations shown in Tables 1 to 3 were prepared. The compositions were prepared by mixing the respective raw material components. The pH of the prepared compositions was 3.8 to 6.9.

The main raw materials used in examples are as follows. Pavlova sp.-derived extracellular vesicle-containing liquid: The sample prepared in the production example was used as it was. Human stem cell-derived extracellular vesicle-containing liquid (product name: RemyStem-JP, manufacturer: Anti-Ageing Co., Ltd) (7.28 × 10⁸ particles/mL) Lactic acid bacteria-derived extracellular vesicle-containing liquid (Extracellular Vesicles derived from Lactobacillus paracasei 180913-R1 strain, manufacturer Cosmo Bio Co., Ltd.) (3.9 × 10¹⁰ particles/mL)

### [Evaluation of stability of particle size and particle count]

For each composition, the state immediately after preparation and the state after standing at 40°C for 1 week were evaluated. That is, the particle count and particle diameter of extracellular vesicles in the composition were measured. The particle diameter was measured using NanoSight, and each composition was appropriately diluted with purified water prior to measurement. With respect to the obtained particle count and particle diameter, the particle count reduction rate and particle size variation rate were calculated using the following formulae. 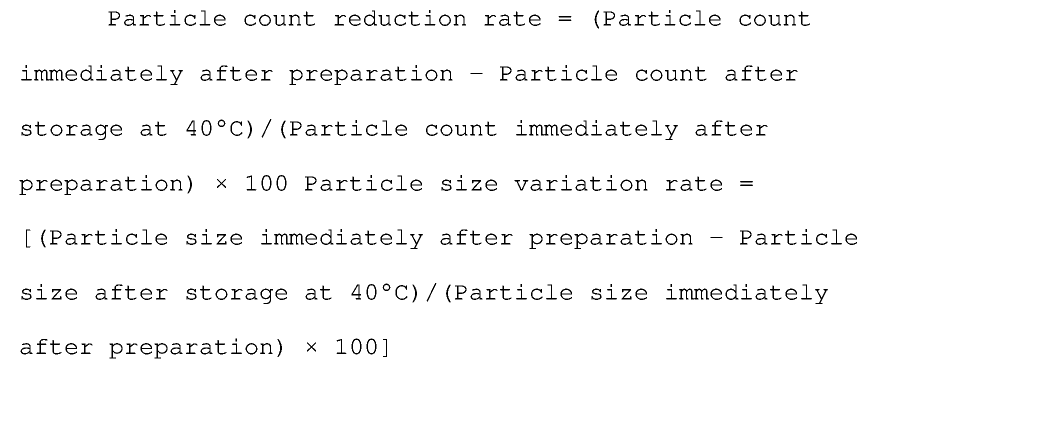 Particle count reduction rate = (Particle count immediately after preparation - Particle count after storage at 40°C)/(Particle count immediately after preparation) × 100 Particle size variation rate = [(Particle size immediately after preparation - Particle size after storage at 40°C)/(Particle size immediately after preparation) × 100]

Note that [ ] denotes the absolute value.

The evaluation results of the compositions of examples and comparative examples are shown in Tables 1 to 3. The numerical values in the tables represent mass%. The evaluation results are shown as follows.

### <Particle count number reduction rate>

20% or more ×
14% or more and less than 20% △
5% or more and less than 14% ○
less than 5% ⊙

### <Particle size variation rate>

Absolute value of 20% or more ×
Absolute value of 10% or more and less than 20% △
Absolute value of less than 10% ○

**[Table 1]**

| | Example 1 | Example 2 | Example 3 | Example 4 | Comparative Example 1 | Comparative Example 2 |
|---|---|---|---|---|---|---|
| *Pavlova* sp.-derived extracellular vesicles | 2 | 2 | 2 | 2 | - | - |
| Human-derived extracellular vesicles | - | - | - | - | 10 | - |
| Lactic acid bacteria-derived extracellular vesicles | - | - | - | - | | 0.187 |
| 1,3-Butylene glycol | - | 10 | - | - | 10 | 10 |
| 1,3-Propanediol | - | - | 10 | - | - | - |
| Dipropylene glycol | - | - | - | 10 | - | - |
| Ethanol | - | - | - | - | - | - |
| Glycerin | 49 | - | - | - | - | - |
| Water | 49 | 88 | 88 | 88 | 80 | 89.813 |
| Total | 100 | 100 | 100 | 100 | 100 | 100 |
| EV count reduction rate (40°C 1 W) | ⊙ | ⊙ | ⊙ | ⊙ | ⊙ | × |
| Particle size variation rate (40°C 1 W) | ○ | ○ | ○ | ○ | × | Δ |

**[Table 2]**

| | Example 5 | Example 6 | Example 7 | Example 8 | Example 9 | Comparative Example 3 | Comparative Example 4 |
|---|---|---|---|---|---|---|---|
| *Pavlova* sp.-derived extracellular vesicles | 2 | 2 | 2 | 2 | 2 | - | - |
| Human-derived extracellular vesicles | - | - | - | - | - | 10 | - |
| Lactic acid bacteria-derived extracellular vesicles | | - | - | - | - | - | 0.187 |
| Dipropylene glycol | - | 10 | - | - | - | - | - |
| Ethanol | 10 | - | 10 | - | - | 10 | 10 |
| Glycerin | - | - | 10 | - | - | - | - |
| PEG-400 | - | - | - | 10 | - | - | - |
| Pentanediol | - | - | - | | 10 | - | - |
| Citric acid | - | 0.1 | - | - | - | - | - |
| Sodium citrate | - | 0.1 | - | - | - | - | - |
| Water | 88 | 87.8 | 78 | 88 | 88 | 80 | 89.813 |
| Total | 100 | 100 | 100 | 100 | 100 | 100 | 100 |
| EV count reduction rate (40°C 1 W) | ○ | ⊙ | ⊙ | ⊙ | ○ | Δ | × |
| Particle size variation rate (40°C 1 W) | ○ | ○ | ○ | ○ | ○ | ○ | × |

**[Table 3]**

| | Example 10 | Example 11 |
|---|---|---|
| *Pavlova* sp.-derived extracellular vesicles | 2 | 2 |
| Human-derived extracellular vesicles | - | - |
| Lactic acid bacteria-derived extracellular vesicles | | |
| Dipropylene glycol | - | - |
| Ethanol | - | - |
| Glycerin | - | - |
| PEG-400 | - | - |
| Pentanediol | - | - |
| Citric acid | 0.3 | - |
| Sodium citrate | 0.2 | - |
| Disodium edetate | - | 0.1 |
| Water | 97.5 | 97.9 |
| Total | 100 | 100 |
| EV count reduction rate (40°C 1 W) | ⊙ | ○ |
| Particle size variation rate (40°C 1 W) | ○ | ○ |

As shown in the tables, extracellular vesicles derived from Pavlova sp. showed little change in both particle count and particle diameter before and after storage. The extracellular vesicles derived from Pavlova sp. were stable even when the component to serve as the base was added. In contrast, extracellular vesicles derived from humans and extracellular vesicles derived from lactic acid bacteria showed variations in particle count and particle diameter before and after storage.

Furthermore, all of the compositions of examples had a smooth texture, excellent moisturizing feel, and good usability.

## Claims

1. A composition comprising microalgae-derived extracellular vesicles (A).

2. A composition comprising:
microalgae-derived extracellular vesicles (A); and
one type or two or more types selected from a group consisting of (B-1) to (B-3):
(B-1) one type or two or more types selected from a group consisting of a primary alcohol and a polyhydric alcohol;
(B-2) one type or two or more types selected from a group consisting of an organic acid and a salt thereof; and
(B-3) a chelating agent.

3. A composition comprising microalgae-derived extracellular vesicles (A) (excluding an anti-aging composition).

4. The composition according to claim 1 or 2, further comprising water.

5. The composition according to claim 1 or 2,
wherein the microalgae are microalgae belonging to a genus Pavlova, Euglena, Spirulina, or Chlorella.

6. The composition according to claim 1 or 2, which is for internal use or external use.

7. A method for improving stability of microalgae-derived extracellular vesicles (A) by allowing one type or two or more types selected from a group consisting of (B-1) to (B-3) to coexist with the microalgae-derived extracellular vesicles:
(B-1) one type or two or more types selected from a group consisting of a primary alcohol and a polyhydric alcohol;
(B-2) one type or two or more types selected from a group consisting of an organic acid and a salt thereof; and
(B-3) a chelating agent.
